# FASCICULE DE BREVET EUROPEEN

(11) **EP 0 649 650 B1**
(45) Date de publication et mention de la délivrance du brevet: **06.12.2000**
(21) Numéro de dépôt: 94402060.1
(22) Date de dépôt: 15.09.1994
(51) Int. Cl.: A61K 9/20, A61K 9/00

(54) **Composition pharmaceutique bioadhésive pour la libération contrôlée de principes actifs**
Bioadhesive pharmazeutische Zusammensetzung zur kontrollierten Freigabe von Wirkstoffen
Bioadhesive pharmaceutical composition for controlled release of drugs

(30) Priorité: 22.09.1993 FR 9311259
(43) Date de publication de la demande: 26.04.1995
(73) Titulaire: ADIR ET COMPAGNIE, 92415 Courbevoie Cédex (FR)
(72) Inventeur: Rault, Isabelle, F-45170 Saint Lye La Foret (FR); Pichon, Gérald, F-45100 Orleans (FR); Cuine, Alain, F-45800 Saint Jean de Braye (FR)

(56) Documents cités:
- WO-A-93/02662
- US-A- 4 948 580
- DATABASE WPI Week 8905, Derwent Publications Ltd., London, GB; AN 89-037180 & JP-A-63 311 960 (SEKISUI CHEM. IND. KK)
- DATABASE WPI Week 8610, Derwent Publications Ltd., London, GB; AN 86-065704 & JP-A-61 017 510 (TOYOBO KK)

## Description

La présente invention concerne une nouvelle composition pharmaceutique bioadhésive permettant la libération contrôlée de principes actifs de manière locale, à travers la cavité buccale ou de manière systémique à travers une muqueuse buccale (jugale ou gingivale), perlinguale, nasale, vaginale ou rectale. La composition pharmaceutique selon l'invention assure une libération plus ou moins rapide du principe actif et peut rester fixée pendant un temps plus ou moins long sur la muqueuse buccale (muqueuse jugale et gencive), perlinguale, nasale, vaginale et rectale.

L'administration par voie transmucosale présente l'avantage au plan métabolique d'éviter la métabolisation importante du principe actif par effet de premier passage hépatique et donc au plan clinique de diminuer les doses administrées en améliorant l'efficacité clinique. Le principe actif ne subit pas les différentes dégradations enzymatiques ou chimiques présentes tout au long du tractus gastro-intestinal, de même que les inconvénients liés à la fonction et à la physiologie de l'appareil gastro-intestinal.

Les possibilités d'administration d'un principe actif à travers une muqueuse dépendent de différents facteurs. En particulier, la composition ne doit pas altérer le tissu d'une manière quelconque à la suite d'un contact prolongé et provoquer des irritations, allergies ou sensibilisations et le principe actif doit pouvoir traverser une surface de tissu assez petite à un débit de diffusion suffisant pour l'obtention de taux plasmatiques adaptés aux besoins thérapeutiques.

Une forme bioadhésive présente la propriété d'adhérer à un tissu biologique, par exemple à une muqueuse dans la cavité buccale, et de s'y maintenir pendant un temps plus ou moins long. Le phénomène de bioadhésion est décrit dans la littérature et est assuré par l'établissement de liaison entre un ou des composés de la forme galénique et des groupements chimiques fonctionnels présents à la surface du tissu biologique. Les interactions intervenant dans le mécanisme de bioadhésion sont décrites comme étant de nature physique, mécanique ou chimique.

Il est connu des documents WO 93/02662 et US 4,938,580 que le copolymère de polyméthylvinyléther et de l'anhydride maléique permet la bioadhésion des compositions pharmaceutiques. La composition pharmaceutique selon l'invention, outre le fait qu'elle soit nouvelle, permet d'obtenir un effet bioadhésif intense et une libération contrôlée et reproductible du principe actif.

La composition pharmaceutique bioadhésive de la présente invention se compose :
- d'un principe actif,
- d'un composé (A) comprenant un ou plusieurs copolymères formés par la copolymérisation du méthylvinyléther et de l'anhydride maléique (ou ses dérivés : acides, esters, sels),
- d'un composé (B) comprenant de l'amidon de maïs modifié et éventuellement un ou plusieurs composés comme la polyvinylpyrrolidone, l'alcool polyvinylique, le polyéthylène glycol, l'acide alginique et alginates dérivés, la cellulose et ses dérivés comme par exemple, l'éthylcellulose, l'hydroxyméthylcellulose, l'hydroxypropylméthylcellulose, etc..., la gomme arabique, la gomme adragante, la gomme guar, la gomme xanthane, le caroube, les carraghénates, les cyclodextrines ou dérivés tels que βcyclodextrines, hydroxypropylβ cyclodextrines, βcyclodextrines partiellement méthylées,
- et, d'excipients jouant le rôle de diluant comme le lactose ou le dihydrogénophosphate dicalcique, de liant, de lubrifiant comme le stéarate de magnésium ou la silice colloïdale, auxquels on peut ajouter des excipients jouant le rôle de promoteurs d'absorption, de colorant, d'aromatisant, d'édulcorant ou d'agents de délitement.

Le composé (A) comprend un ou plusieurs copolymères sélectionnés parmi des copolymères du méthylvinyléther et de l'anhydride maléique ou de ses dérivés sous forme d'acides, d'esters ou de sels. Ces copolymères possèdent la formule suivante :

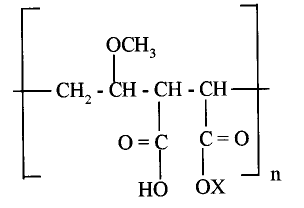

Des exemples précis de ce type de copolymères actuellement disponibles dans le commerce sont les GANTREZ® de type AN, S, ES et MS (GAF products).
- GANTREZ AN® = anhydride
- GANTREZ S® = acide, X = H
- GANTREZ ES® = ester, X = alkyl
- GANTREZ MS® = sel, X = Ca ou Na

La bioadhésion est assurée principalement par le composé (A) et la libération prolongée est assurée par l'association du composé (A) et du composé (B). La proportion du composé (A) est entre 5 et 85 % en masse et la proportion du composé (B) est entre 5 et 85 % en masse. Selon les proportions utilisées, la bioadhésion et la libération prolongée peuvent être modulées.

Le composé (A) est préférentiellement un mélange des sels de sodium et de calcium du copolymère de méthylvinyléther et de l'anhydride maléique (ou ses dérivés: acides, esters, sels). La quantité de composé (A) se situe préférentiellement entre 10 et 30 % en masse. La quantité de composé (B) se situe préférentiellement entre 10 et 50 % en masse. Le diluant peut être le lactose, le dihydrogénophosphate de calcium ou d'autres diluants appropriés.

La composition pharmaceutique selon l'invention peut se présenter sous la forme d'un comprimé, d'une pastille, d'une crème ou d'un gel. Une forme préférentielle est l'obtention d'un comprimé qui peut être préparé par le mélange du composé (A), du composé (B), du principe actif et des excipients par granulation humide suivie d'une compression.

Dans le procédé par granulation, les deux polymères sont mis en oeuvre séparément dans le comprimé grâce à un procédé de fabrication original qui associe une phase interne granulée et une phase externe non granulée. Ce procédé permet de conserver intactes les propriétés bioadhésives du polymère de départ et de contrôler, de manière reproductible, la bioadhésion et la libération du principe actif.

Ce procédé par granulation se compose de différentes étapes successives :
La préparation de la phase interne s'effectue par le mélange du principe actif avec tout ou partie de la quantité du composé (B) auxquels est ajouté le diluant.
Le mélange obtenu est ensuite granulé avec un liquide de mouillage, préférentiellement de l'eau ou des mélanges hydroalcooliques.
Le granulé obtenu est tamisé puis séché à une température se situant entre 20 et 100°C. Le granulé est alors calibré par passage sur un tamis.
Parallèlement, la phase dite externe est réalisée. Elle se compose du mélange du composé (A) et du composé (B) s'il n'a pas été introduit totalement en phase interne.
Les phases interne et externe sont ensuite mélangées. On ajoute éventuellement à ce mélange les excipients définis comme jouant le rôle de lubrifiant, promoteurs d'absorption, aromatisant, édulcorant, colorant et délitant. L'ensemble est mélangé puis calibré par un passage sur un tamis.
Le mélange obtenu est comprimé.

Le choix de la forme et de la taille du comprimé est important. Ces deux critères conditionnent la cinétique de libération souhaitée, influent sur la bioadhésion en modifiant la surface de contact entre la muqueuse et le forme galénique et enfin rendent plus agréable la pose et le maintien du comprimé.

La composition pharmaceutique selon l'invention peut se présenter sous la forme d'un comprimé rond de 3 à 15 mm de diamètre et de 1 à 5 mm d'épaisseur. Elle peut également se présenter par exemple sous la forme d'un comprimé hémi-convexe, oblong, rond chanfreiné ou toute autre forme géométrique présentant l'intérêt d'assurer le meilleur maintien prolongé de la forme par exemple dans le sillon maxillo-gingivale de la cavité buccale ou sous la langue.

La composition pharmaceutique selon l'invention est caractérisée en ce qu'elle se maintient appliquée sur la muqueuse buccale, perlinguale, nasale, rectale ou vaginale pour une durée allant de 10 minutes jusqu'à 12 heures.

Parmi les principes actifs entrant dans la composition selon l'invention, on peut citer, à titre non limitatif, les agents antiinflammatoires et les analgésiques qu'ils soient à action locale ou systémique comme par exemple l'indométacine, l'ibuprofène, le diclofénac, le tenoxicam, le piroxicam, les désinfectants comme la chlorhexidine et les sels dérivés, les enzymes, les vasodilatateurs coronariens comme la nitroglycérine ou le dinitrate d'isosorbide, les antiasthmatiques, les antibactériens, les antibiotiques comme la pénicilline ou l'érythromycine, les cardiotoniques comme la digitaline, les anesthésiques locaux comme la lidocaïne, les antiangoreux, les antidysrythmiques, les antihypertenseurs, les antiagrégants, les antitussifs et les expectorants comme le phosphate de codéine, les antihistaminiques comme la chlorphénamine, les antiinflammatoires stéroidiens comme la prednisolone, les agonistes dopaminergiques comme le piribédil, les régulateurs du sommeil comme la mélatonine, les agents favorisant l'hémostase, les hormones, les agents antitumoraux, antimigraineux, antiparkinsoniens, promnésiants, les antidépresseurs, les anxiolytiques, les hypnotiques, les antidiabétiques, les agents antiobésité, les antifungiques, les antiviraux.
Les exemples suivants illustrent l'invention mais ne la limitent en aucune façon.

### EXEMPLE 1:

La fabrication des comprimés fait appel à un procédé dit de granulation humide. La préparation de la phase interne s'effectue par le mélange de 320 grammes de piribédil avec 80 grammes d'amidon de maïs modifié (CERESTAR TOP SF12616, société CERESTAR) auxquels sont ajoutés 278 grammes de dihydrogénophosphate de calcium. Le mélange est réalisé à l'aide d'un Turbula pendant 15 minutes. Le mélange obtenu est ensuite placé dans un second mélangeur de type planétaire, l'eau est ajoutée progressivement sous agitation, à ce mélange. Le mélange est ensuite tamisé sur un tamis de 1,25 mm d'ouverture de maille. La poudre obtenue est séchée à une température de 60°C pendant une heure puis tamisée sur un tamis de 0,80 mm d'ouverture de maille. Parallèlement, la phase dite externe est réalisée. Elle se compose du mélange de 150 grammmes de GANTREZ MS 955 et de 160 grammes d'amidon de maïs modifié (CERESTAR TOP SF12616). Le mélange est effectué à l'aide d'un mélangeur par retournement Turbula pendant 15 minutes. Les phases interne et externe sont ensuite mélangées à l'aide d'un mélangeur par retournement Turbula. On ajoute ensuite à ce mélange 10 grammes de stéarate de magnésium et 2 grammes de silice colloïdale. L'ensemble est mélangé à l'aide d'un mélangeur par retournement Turbula. Le mélange obtenu est calibré par un passage sur un tamis de 0,4 mm d'ouverture de maille.

### EXEMPLE 2:

La fabrication des comprimés fait appel à un procédé dit de granulation humide. La préparation de la phase interne s'effectue par le mélange de 320 grammes de piribédil avec 80 grammes d'amidon de maïs modifié (CERESTAR TOP SF12616) auxquels sont ajoutés 278 grammes de lactose (Lactose Fast Flo, société SEPPIC). Le mélange est réalisé à l'aide d'un Turbula pendant 15 minutes. Le mélange obtenu est ensuite placé dans un second mélangeur par retournement, l'eau est ajoutée progressivement sous agitation, à ce mélange. Le mélange est ensuite tamisé sur un tamis de 1,25 mm d'ouverture de maille. La poudre obtenue est séchée à une température de 60°C pendant une heure puis tamisée sur un tamis de 0,80 mm d'ouverture de maille. Parallèlement, la phase dite externe est réalisée. Elle se compose du mélange de 150 grammes de GANTREZ MS 955 et de 160 grammes d'amidon de maïs modifié (CERESTAR TOP SF12616). Le mélange est effectué à l'aide d'un mélangeur par retournement Turbula pendant 15 minutes. Les phases interne et externe sont ensuite mélangées à l'aide d'un mélangeur par retournement Turbula. On ajoute ensuite à ce mélange 10 grammes de stéarate de magnésium et 2 grammes de silice colloïdale. L'ensemble est mélangé à l'aide d'un mélangeur par retournement Turbula. Le mélange obtenu est calibré par un passage sur un tamis de 0,4 mm d'ouverture de maille.

### EXEMPLE 3:

La fabrication des comprimés fait appel à un procédé dit de granulation humide. La préparation de la phase interne s'effectue par le mélange de 166,5 grammes de piribédil avec 80 grammes d'amidon de maïs modifié (CERESTAR TOP SF 12616, société CERESTAR) auxquels sont ajoutés 373 grammes de dihydrogénophosphate de calcium. Le mélange est réalisé à l'aide d'un mélangeur par retournement de type OURS VARYMIXER. 66,5 grammes de copolymère d'éthylacrylate et de méthylmétacrylate en solution aqueuse à 30 % en poids sec sont ajoutés progressivement sous agitation. La quantité de liquide de mouillage nécessaire à la granulation humide est obtenue par addition d'eau purifiée. Le mélange obtenu est ensuite passé dans un granulateur de type FREWITT sur une grille de 2 mm. Le mélange obtenu est alors séché dans une étuve ventilée à 60°C pendant deux heures. Le mélange est ensuite passé dans un granulateur de type FREWITT sur une grille de 0,7 mm. Parallèlement, la phase dite externe est réalisée. Elle se composé du mélange de 160 grammes de GANTREZ MS 955 (société ISP) et de 147 grammes d'hydroxypropylméthylcellulose (HPMC K 100M P, COLORCON). Le mélange des deux phases est effectué dans un mélangeur par retournement de type RHON. On ajoute les lubrifiants à ce mélange : 5 grammes de stéarate de magnésium et 2 grammes de silice colloïdale préalablement tamisés sur 0,4 mm. L'ensemble est mélangé dans un mélangeur par retournement de type RHON. Le grain obtenu est ensuite comprimé.

### EXEMPLE 4:

Selon le même procédé que l'exemple 1, les 320 grammes de piribédil sont remplacés par 32 grammes de mélatonine et la quantité de dihydrogénophosphate dicalcique est de 566 grammes. Le reste est inchangé.

### EXEMPLE 5:

Selon le même procédé que l'exemple 1, les 320 grammes de piribédil sont remplacés par 32 grammes de lidocaïne et la quantité de dihydrogénophosphate dicalcique est de 566 grammes. Le reste est inchangé.

### EXEMPLE 6:

La fabrication des comprimés fait appel à un procédé dit de granulation humide. La préparation de la phase interne s'effectue par le mélange de 333 g de chlorhydrate d'amineptine avec 79 grammes d'amidon de maïs modifié (CERESTAR TOP SF 12616, société CERESTAR) auxquels sont ajoutés 260 grammes de dihydrogénophosphate de calcium. Le mélange est réalisé à l'aide d'un mélangeur par retournement de type OURS VARYMIXER. L'eau est ajoutée progressivement sous agitation et le mélange est ensuite passé dans un granulateur de type FREWITT sur une grille de 2 mm. La poudre obtenue est alors séchée dans une étuve ventilée à 60°C pendant deux heures. Le mélange est ensuite passé dans un granulateur de type FREWITT sur une grille de 0,7 mm. Parallèlement, la phase dite externe est réalisée. Elle se compose du mélange de 159 grammes de GANTREZ MS 955 (société ISP) et de 157 grammes d'hydroxypropylméthylcellulose (HMPC K 100M P, société COLORCON). Le mélange des deux phases est effectué dans un mélangeur à retournement de type RHON. On ajoute les lubrifiants à ce mélange : 10 grammes de stéarate de magnésium et 2 grammes de silice colloïdale préalablement tamisés sur 0,4 mm. L'ensemble est mélangé dans un mélangeur par retournement de type RHON. Le grain obtenu est ensuite comprimé.

### EXEMPLE 7:

La fabrication des comprimés fait appel à un procédé dit de granulation humide. La préparation de la phase interne s'effectue par le mélange de 145 grammes de chlorhydrate de 1,3,7-triméthyl-8-{3-[4-(diéthylaminocarbonyl)pipérazin-1-yl]prop-1-yl}-3,7-dihydro(1H) purine-2,6-dione (décrit dans le brevet EP 149578) avec 79,5 grammes d'amidon de maïs modifié (CERESTAR TOP SF 12616, société CERESTAR) auxquels sont ajoutés 346 grammes de dihydrogénophosphate de calcium. Le mélange est réalisé à l'aide d'un mélangeur par retournement de type OURS VARYMIXER. L'eau est ajoutée progressivement sous agitation et le mélange est ensuite passé dans un granulateur de type FREWITT sur une grille de 2 mm. La poudre obtenue est alors séchée dans une étuve ventilée à 60°C pendant deux heures. Le mélange est ensuite passé dans un granulateur de type FREWITT sur une grille de 0,7 mm. Parallèlement, la phase dite externe est réalisée. Elle se compose du mélange de 159 grammes de GANTREZ MS 955 (société ISP), de 262,5 grammes d'hydroxypropylméthylcellulose (HPMC K 100M P, COLORCON) et 1 gramme de saccharinate de sodium. Le mélange des deux phases est effectué dans un mélangeur par retournement de type RHON. On ajoute les lubrifiants à ce mélange : 5 grammes de stéarate de magnésium et 2 grammes de silice colloïdale préalablement tamisés sur 0,4 mm. L'ensemble est mélangé dans un mélangeur par retournement de type RHON. Le grain obtenu est ensuite comprimé.

## Revendications

1. Composition pharmaceutique bioadhésive pour la libération contrôlée d'un principe actif de manière locale dans la cavité buccale ou de manière systémique a travers une muqueuse comprenant :
- un principe actif,
- un compose (A) constitué par un ou plusieurs copolymères formés par la copolymérisation du méthylvinyléther et de l'anhydride maléique ou de ses dérivés,
- un compose (B) constitué par de l'amidon de maïs modifié et éventuellement un ou plusieurs composes choisis parmi la polyvinylpyrrolidone, l'alcool polyvinylique, le polyéthylène glycol, l'acide alginique et ses dérivés, la cellulose et ses dérivés, la gomme arabique, la gomme adragante, la gomme guar, la gomme xanthane, le caroube, les carraghénates, les cyclodextrines et leurs dérivés,
- et, d'excipients thérapeutiquement acceptables.

2. Composition pharmaceutique selon la revendication 1 caractérisée en ce que le composé (A) est constitué par un ou plusieurs copolymères de méthylvinyléther et d'anhydride maléique ou de ses dérivés sous forme d'acides, d'esters ou de sets de calcium ou de sodium.

3. Composition pharmaceutique selon la revendication 1 caractérisée en ce que la quantité du compose (A) est comprise entre 5 et 85 % de ta masse totale de la composition.

4. Composition pharmaceutique selon la revendication 1 caractérisée en ce que la quantité du compose (B) est comprise entre 5 et 85 % de la masse totale de la composition.

5. Composition pharmaceutique selon la revendication 1 caractérisé en ce que le principe actif est un agent antiinflammatoire ou un analgésique qu'il soit à action locale ou systémique, un désinfectant, une enzyme, un vasodilatateur coronarien, un antiasthmatique, un antibactérien, un antibiotique, un cardiotonique, un anesthésique local, un antiangoreux, un antidysrythmique, un antihypertenseur, un antiagrégant, un antitussif, un expectorant, un antihistaminique, un agoniste dopaminergique, un régulateur du sommeil, un hémostatique, une hormone, un antitumoral, un antimigraineux, un antiparkinsonien, un promnésiant, un antidépresseur, un anxiolytique, un hypnotique, un antidiabétique, un antiobésité, un antifungique ou un antiviral.

6. Composition pharmaceutique selon la revendication 1 caractérisée en ce que les excipients sont choisis dans le groupe des diluants, des liants, des lubrifiants auxquels on peut ajouter des colorants, des aromatisants, des édulcorants, des délitants ou des promoteurs d'absorption.

7. Composition pharmaceutique selon la revendication 1 caractérisée en ce quelle se présente sous la forme d'un comprimé.

8. Comprimé selon la revendication 7 caractérisé en ce qu'il est obtenu par granulation humide suivie d'une compression.

9. Procédé de préparation du comprimé selon la revendication 7 caractérisé en ce qu'il est obtenu par compression d'un mélange constitué :
- dune phase interne obtenue par mélange du principe actif, de tout ou partie du composé (B) et d'un diluant suivie dune granulation humide,
- dune phase externe obtenue par mélange du compose (A) et du compose (B) lorsque celui-ci n'a pas été introduit en totalité dans la phase interne,
- éventuellement, d'excipients appropriés pharmaceutiquement acceptables.

## Claims

1. Bioadhesive pharmaceutical composition for the controlled release of an active ingredient locally in the buccal cavity or systemically through a mucous membrane, comprising:
- an active ingredient,
- a component (A) consisting of one or more copolymers formed by the copolymerisation of methyl vinyl ether and maleic anhydride or its derivatives,
- a component (B) consisting of modified corn starch and, optionally, one or more components selected from polyvinylpyrrolidone, polyvinyl alcohol, polyethylene glycol, alginic acid and its derivatives, cellulose and its derivatives, gum arabic, gum tragacanth, guar gum, xanthan gum, carob, carrageenates, cyclodextrins and their derivatives, and
- therapeutically acceptable excipients.

2. Pharmaceutical composition according to claim 1, characterised in that component (A) consists of one or more copolymers of methyl vinyl ether and maleic anhydride or its derivatives in the form of acids, esters or calcium or sodium salts.

3. Pharmaceutical composition according to claim 1, characterised in that the amount of component (A) is from 5 to 85 % of the total weight of the composition.

4. Pharmaceutical composition according to claim 1, characterised in that the amount of component (B) is from 5 to 85 % of the total weight of the composition.

5. Pharmaceutical composition according to claim 1, characterised in that the active ingredient is an anti-inflammatory or an analgesic acting either locally or systemically, a disinfectant, an enzyme, a coronary vasodilator, an anti-asthma agent, an antibacterial, an antibiotic, a cardiotonic, a local anaesthetic, an antianginal, an antiarrhythmic, an antihypertensive, an anti-aggregating agent, an antitussive, an expectorant, an antihistamine, a dopaminergic agonist, a sleep regulator, a haemostatic, a hormone, an antitumour agent, an antimigraine agent, an anti-Parkinsonism agent, a pro-mnesic agent, an antidepressant, an anxiolytic, a hypnotic, an anti-diabetic, an anti-obesity agent, an antifungal or an antiviral.

6. Pharmaceutical composition according to claim 1, characterised in that the excipients are selected from the group of the diluents, the binders, the lubricants, to which there may be added cotourings, flavourings, sweeteners, disintegrators or absorption promoters.

7. Pharmaceutical composition according to claim 1, characterised in that it is in the form of a tablet.

8. Tablet according to claim 7, characterised in that it is obtained by wet granulation followed by compression.

9. Process for the preparation of the tablet according to claim 7, characterised in that it is obtained by compressing a mixture consisting of:
- an inner phase obtained by mixing the active ingredient, all or part of component (B) and a diluent, followed by wet granulation,
- an outer phase obtained by mixing component (A) and component (B), when all of the latter has not been introduced into the inner phase,
- optionally, suitable pharmaceutically acceptable excipients.

## Patentansprüche

1. Bioadhäsive pharmazeutische Zubereitung zur kontrollierten Freisetzung eines Wirkstoffs in lokaler Weise in der Mundhöhle oder in systemischer Weise über eine Schleimhaut, umfassend:
- einen Wirkstoff,
- eine Verbindung (A), die durch eines oder mehrere Copolymere gebildet ist, die durch Copolymerisation von Methylvinylether und Maleinsäureanhydrid oder ihren Derivaten hergestellt worden sind,
- eine Verbindung (B), die aus modifizierter Maisstärke und gegebenenfalls einer oder mehreren Verbindungen ausgewählt aus Polyvinylpyrrolidon, Polyvinylalkohol, Polyethylenglykol, Alginsäure und deren Derivaten, Cellulose und deren Derivaten, Gummi arabicum, Tragantgummi, Guargummi, Xanthangummi, Johannisbrotkernmehl, Carragheenaten, Cyclodextrinen und deren Derivaten ausgewählt ist,
- und therapeutisch annehmbare Trägermaterialien.

2. Pharmazeutische Zubereitung nach Anspruch 1, dadurch gekennzeichnet, daß die Verbindung (A) aus einem oder mehreren Copolymeren aus Methylvinylether und Maleinsäureanhydrid oder einem ihrer Derivate in Form von Säuren, Estern oder Calcium- oder Natriumsalzen gebildet ist.

3. Pharmazeutische Zubereitung nach Anspruch 1, dadurch gekennzeichnet, daß die Menge der Verbindung (A) zwischen 5 und 85 % der Gesamtmasse der Zubereitung liegt.

4. Pharmazeutische Zubereitung nach Anspruch 1, dadurch gekennzeichnet, daß die Menge der Verbindung (B) zwischen 5 und 85 % der Gesamtmasse der Zubereitung liegt.

5. Pharmazeutische Zubereitung nach Anspruch 1, dadurch gekennzeichnet, daß der Wirkstoff ein antiinflammatorisches oder analgetisches Mittel mit entweder lokaler oder systemischer Wirkung, ein Desinfektionsmittel, ein Enzym, ein Koronargefäßdilatator, ein Antiasthmatikum, ein antibakterielles Mittel, ein Antibiotikum, ein Kardiotonikum, ein Lokalanästhetikum, ein Antianginamittel, ein Antidysrhythmisches Mittel, ein Antihypertensivum, ein antiaggregierendes Mittel, ein Hustenmittel, ein Expektorans, ein Antihistaminikum, ein dopaminergischer Agonist, ein Schlafregulierungsmittel ein Hämostatikum, ein Hormon, ein Antitumormittel, ein Antimigränemittel ein Antiparkinsonmittel, ein Gedächtnisfördernd, ein Antidepressivum, ein Anxiolytikum, ein Hypnotikum, ein Antidiabetikum, ein gegen die Fettsucht gerichtetes Mittel, ein antifungales oder antivirales Mittel ist.

6. Pharmazeutische Zubereitung nach Anspruch 1, dadurch gekennzeichnet, daß die Trägermaterialien aus der Gruppe der Verdünnungsmittel, der Bindemittel, Der Schmiermittel, denen man Farbstoffe zusetzen kann, der Aromatisierungsmittel, der Süßungsmittel, der Sprengmittel oder Absorptionspromotoren ausgewählt sind.

7. Pharmazeutische Zubereitung nach Anspruch 1, dadurch gekennzeichnet, daß sie in Form einer Tablette vorliegt.

8. Tablette nach Anspruch 7, dadurch gekennzeichnet, daß sie durch Feuchtgranulation, gefolgt von einem Verpressen, hergestellt worden ist.

9. Verfahren zur Herstellung der Tablette nach Anspruch 7, dadurch gekennzeichnet, daß man sie durch Verpressen der folgenden Mischung bildet:
- eine interne Phase, die durch Vermischen des Wirkstoffs mit der Gesamtmenge oder einem Teil der Verbindung (B) und einem Verdünnungsmittel, gefolgt von einer Feuchtgranulation, erhalten worden ist,
- einer externen Phase, die durch Vermischen der Verbindung (A) und der Verbindung (B), falls diese noch nicht vollständig in die interne Phase eingebracht worden ist, gebildet worden ist,
- gegebenenfalls geeignete pharmazeutisch annehmbare Trägermaterialien.
